# EUROPÄISCHE PATENTANMELDUNG

(11) **EP 1 312 389 A1**
(43) Veröffentlichungstag der Anmeldung: **21.05.2003**
(21) Anmeldenummer: 02025701.0
(22) Anmeldetag: 15.11.2002
(51) Int. Cl.: A61M 5/32

(54) **Vorrichtung zur Aufnahme von benutzten Punktionsnadeln oder Injektionsnadeln**

(30) Priorität: 20.11.2001 DE 10156826
(71) Anmelder: Schwarzpunkt Schwarz GmbH & Co., 82205 Gilching (DE)
(72) Erfinder: Schwarz, Ingo, 82205 Gilching (DE); Schmidt, Manfred, 86807 Buchloe (DE)
(74) Vertreter: Zmyj, Erwin, Dipl.-Ing., Dipl.-Wirtsch.-Ing., European Patent Attorney

(57) **Zusammenfassung**

Die Vorrichtung zur Aufnahme von benutzten Kanülen umfaßt einen aus einem widerstandsfähigen Material bestehenden Schutzbehälter (2) und einen äußeren, den Schutzbehälter aufnehmenden Standbehälter (3), der an seinem unteren Ende einen als Saugnapf ausgebildeten Standfuß (4) und an seinem oberen Ende einen Verschlußkörper (12) trägt, der über eine Lasche (13) mit dem Standbehälter (3) fest verbunden ist. Der Innenkonus des Schutzbehälters (2) entspricht in seinem Winkel dem Nadelkonus (8), so daß dieser nach dem Einstecken der Injektionsnadel durch Reibschluß in dem Schutzbehälter festgelegt ist. Eine formschlüssige Verriegelung (10,11) ist als zusätzliche Sicherung vorgesehen.

## Beschreibung

Die Erfindung bezieht sich auf eine Vorrichtung zur Aufnahme von benutzten Punktionsnadeln oder Injektionsnadeln.

Punktionsnadeln oder Injektionsnadeln sind nach ihrer Benutzung kontaminiert, so daß man bemüht sein muß grundsätzlich eine Verletzung durch die Nadel oder eine Berührung mit eventuell belastetem Blut (AIDS, Hepatitis etc.) zu vermeiden.

Es ist bekannt, würfelförmige Kunststoffkörper für die Aufnahme der Nadelspitze zur Verfügung zu stellen. Hierbei ist nur ein geringer Schutz gegen Verletzungen zu erzielen, weil dieser Kunststoffkörper leicht durchstochen werden kann. Ein Schutz gegen Berührungen von belastetem Blut besteht hierbei nicht. Weiterhin ist bekannt geworden, die Nadeln in köcherartige Behälter einzuführen. Diese sind aber aus einem weichen Kunststoff gefertigt, so daß ein Durchstechen der Wand dieser Behälter bei schräger Einführung nicht immer vermeidbar ist. Außerdem sind diese Behälter nicht verschließbar.

Aufgabe der Erfindung ist es, eine Vorrichtung zur Aufnahme von Injektionsnadeln oder Punktionsnadeln zu schaffen, die sowohl einen Schutz gegen Verletzung durch die spitze Nadel als auch einen Schutz gegen Berührung mit belastetem Blut gewährleistet.

Diese Aufgabe wird erfindungsgemäß gelöst durch einen einseitig offenen, röhrenförmigen Schutzbehälter aus einem gegen Durchstechen widerstandsfähigen Material mit einem Innenraum zur vollständigen Aufnahme der gesamten Injektionsnadel, der in seinem oberen offenen Bereich einen dem Nadelkonus angepaßten Aufnahmekonus zur reibschlüssigen Festlegung des Nadelkonus aufweist und durch einen den röhrenförmigen Behälter aufnehmenden Standbehälter sowie ferner durch einen Verschlußkörper, der zumindest den Zugang zum Nadelkonus versperrt.

Die erfindungsgemäße Vorrichtung umfaßt somit zwei Teile, nämlich einen Schutzbehälter, der die kontaminierte Nadel mit ihrem Nadelkonus aufnimmt, wobei der Nadelkonus in dem Schutzbehälter so festlegbar ist, daß die Spritze von der Nadel abgezogen werden kann, ohne eine zweite Hand zu benutzen und aus einem Standbehälter, um den Schutzbehälter in einer solchen Form dem Benutzer zu präsentieren, daß dieser die Nadel leicht einführen kann. Weiterhin umfaßt diese Schutzvorrichtung einen Verschlußkörper, der zumindest den Zugang zum Nadelkonus versperrt und somit eine Berührung mit der Punktionsnadel oder der Injektionsnadel verhindert. Da der Schutzbehälter aus einem gegen Durchstechen widerstandsfähigen Material besteht, wird auch bei einem unachtsamen Einführen der Nadel in den Schutzbehälter vermieden, daß dieser Durchstochen wird und somit Anlaß zu Verletzungen geben kann.

Wenn in weiterer Ausgestaltung der Erfindung der Standbehälter den röhrenförmigen Schutzbehälter vollständig aufnimmt, so wird hierdurch die Voraussetzung geschaffen, den Schutzbehälter dünnwandig auszuführen und ihn dabei trotzdem sicher zu halten.

In vorteilhafter Weiterbildung der Erfindung besteht der Standbehälter aus einem weichelastischen Kunststoff und ist durch Umspritzen des Schutzbehälters mit diesem fest verbunden. Eine Trennung des Schutzbehälters vom Standbehälter ist somit nicht möglich. Beide Teile bilden gemeinsam eine Schutzvorrichtung für die gefahrlose Entsorgung benutzter Nadeln. Damit der Verschlußkörper im. Benutzungsfalle stets zur Hand ist, ist in weiterer Ausbildung der Erfindung vorgesehen, daß der Standbehälter über eine angeformte biegsame Lasche den Verschlußkörper trägt. Hierdurch ist dieser immer stets griffbereit vorhanden, wenn die Nadel in den Schutzbehälter eingeführt ist.

Bei einer bevorzugten Ausgestaltung der Erfindung ist der Verschlußkörper in den hohlen Nadelkonus einsteckbar. Hierdurch wird gewährleistet, daß noch möglicherweise in der Nadel vorhandenes Blut nicht nach außen austreten kann.

Der Verschlußkörper kann aber auch als feststeckbarer Deckel für den Standbehälter ausgebildet sein, wodurch er den rückwärtigen Teil des Nadelkonus vollständig überdeckt und damit gegenüber der Umwelt abschließt. Um eine besondere sichere Halterung des Nadelkonus in dem Schutzbehälter zu ermöglichen, ist zusätzlich zu dem Reibschluß, der sich aufgrund des Zusammenwirkens beider konischen Flächen von Nadelkonus und Schutzbehälter ergibt, in weiterer Ausgestaltung der Erfindung vorgesehen, daß der Standbehälter im Bereich seines oberen, offenen Endes mindestens einen Vorsprung für mindestens einen gewindeartigen oder bajonettverschlußartigen Vorsprung am Nadelkonus aufweist. Hierdurch ist der Nadelkonus nach seinem Einführen in den Schutzbehälter durch Drehbewegung formschlüssig mit dem Standbehälter verbindbar.

Damit der Standbehälter bei einer Einhandbedienung einen sicheren Stand aufweist und somit auch ein sicheres Einführen der benutzten Nadel in den Schutzbehälter gewährleisten kann, ist in weiterer vorteilhafter Ausgestaltung der Erfindung vorgesehen, daß der Standbehälter einen saugnapfartigen Standfuß aufweist. Hierdurch ist der Standbehälter auf einer glatten Unterlage durch Andrücken festsaugbar, wodurch er auch bei einem seitlichen Anstoßen nicht umfällt und somit für das Einführen der Nadeln auch bei einer Einhandbedienung stets benutzerfreundlich zur Verfügung steht.

Es ist aber auch möglich, daß der Standbehälter einen unten ebenen Standfuß mit einer Klebebeschichtung aufweist und somit auf seiner Standunterlage festlegbar ist, auch wenn diese für eine Saugnapfbefestigung ungeeignet sein sollte.

Wenn in weiterer vorteilhafter Ausgestaltung der Erfindung der Schutzbehälter eine Behälterwand mit insgesamt konischem Verlauf aufweist, deren Konuswinkel im wesentlichen demjenigen des Nadelkonus entspricht, so wird hierdurch die Herstellung eines solchen Schutzbehälters vereinfacht. Die gleichmäßige konische Ausgestaltung des Schutzbehälters ist unempfindlich gegen Maßtoleranzen, weil dieser Reibschluß durch mehr oder weniger tiefes Einstecken des Nadelkonus in den Schutzbehälter erzielbar ist. Eine solche Ausgestaltung ermöglicht auch eine gleichmäßige verhältnismäßig dünne Behälterwand, was den Aufwand bei der Herstellung vermindert.

Wenn bei einer weiteren vorteilhaften Ausgestaltung der Erfindung der Schutzbehälter an seinem oberen offenen Ende mit einem Außenflansch versehen ist, so wird dieser Schutzbehälter an seinem offenen Ende verstärkt, was das Umspritzen bei der Herstellung des äußeren Standbehälters erleichtert, insbesondere dann, wenn der Außenflansch einen geringfügig größeren Durchmesser aufweist als dies den größten äußeren Abmessungen des Nadelkonus entspricht, weil dann der äußere Standbehälter einen den Schutzbehälter überragenden zylindrischen Teil aufweisen kann, was erforderlich ist, wenn der Standbehälter eine zusätzliche formschlüssige Sicherung in Form einer gewindeähnlichen oder bajonettartigen Verriegelungsmöglichkeit aufweist.

Die Herstellung der Gesamtvorrichtung wird dadurch entscheidend vereinfacht, wenn in Weiterbildung der Erfindung der Schutzbehälter aus einem harten Kunststoff besteht, weil dann die Vorrichtung in einem zweistufigen Spritzverfahren herstellbar ist.

Die Erfindung wird nachstehend anhand eines in der Zeichnung dargestellten Ausführungsbeispieles näher erläutert. In der Zeichnung zeigen:
- **Figur 1:**: eine Seitenansicht einer Vorrichtung für die Aufnahme einer Injektionsnadel;
- **Figur 2:**: einen Schnitt nach der Linie II - II in Figur 1; und
- **Figur 3:**: eine Ansicht von oben auf Figur 1.

Die Vorrichtung zur Aufnahme von benutzten Punktionsnadeln oder Injektionsnadeln, die in der Zeichnung insgesamt mit 1 bezeichnet ist, umfaßt einen Schutzbehälter 2 aus einem gegen Durchstechen widerstandfähigen Material, beispielsweise einem harten Kunststoff und einem Standbehälter 3, der durch Umspritzen des Schutzbehälters 2 mit einem weicheren Kunststoffmaterial hergestellt wird und somit fest mit dem Schutzbehälter verbunden ist. Der Standbehälter weist an seinem unteren Ende einen Fuß 4 auf, der im vorliegenden Falle im Innenbereich der Standfläche 5 einen Hohlraum 6 begrenzt, so daß dieser Fuß 4 als Saugnapf dient und ein Festlegen des Standbehälters auf glatten Flächen ermöglicht.

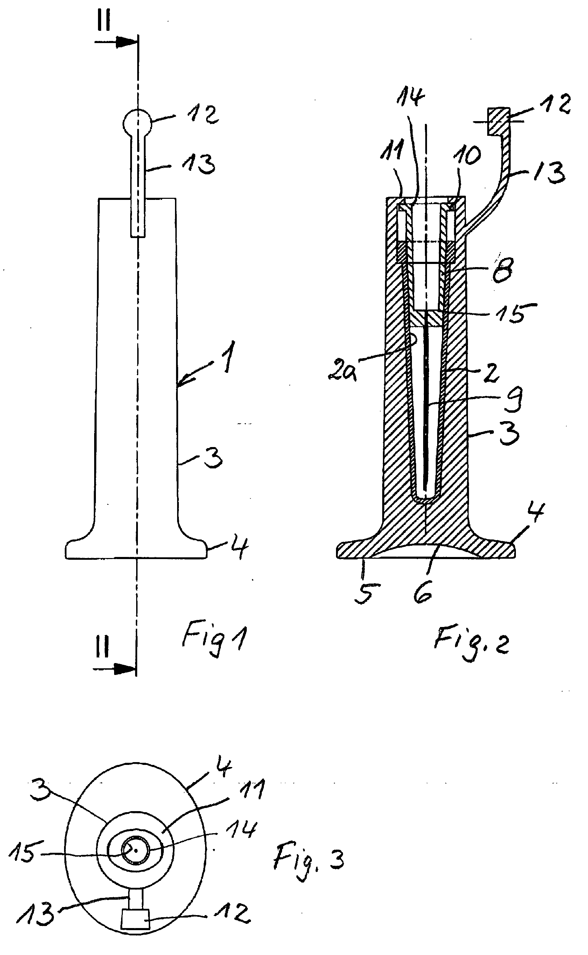

Der Schutzbehälter 2 ist gleichmäßig konisch gestaltet und weist an seinem oberen Rand einen äußeren Verstärkungsflansch 7 auf. Der Innenkonus 2a des Schutzbehälters 2 ist dem Außenkonus des Nadelkonus 8 angepaßt, so daß der Nadelkonus einer Kanüle, d.h. einer Injektions- oder Punktionsnadel durch Einstecken in den Schutzbehälter derart reibschlüssig mit diesem verbindbar ist, daß die Kanüle nicht mehr aus dem Schutzbehälter herausfällt. Der Schutzbehälter weist einen Innenraum auf, der so lang ist, daß er noch genügend Abstand zu der Nadel 9 beläßt, wenn diese in den Schutzbehälter eingeführt ist. Der Nadelkonus 8 weist an seinem oberen Ende Vorsprünge 10 auf, mit denen er hinter bajonettartigen Vorsprüngen 11 des Standbehälters einrastbar ist. Dies ist eine zusätzliche Sicherung der Kanüle in dem Schutzbehälter. Ein mit 12 bezeichneter Stopfen ist über eine Lasche 13 mit dem Standbehälter 3 fest verbunden und dient zum Verschließen des Innenraumes des Nadelkonus 8. Der Nadelkonus weist eine gleichmäßige Wandstärke auf und ist somit auch innen konisch ausgebildet. Die obere Kante ist hierbei mit 14 und die untere Kante des Innenraumes mit 15 bezeichnet. Wie aus der Draufsicht in Figur 3 ersichtlich, ist der Fuß 4 oval ausgeführt.

Damit die Vorsprünge 10 des Nadelkonus unter einen nach innen ragenden umlaufenden Vorsprung 11 bringbar sind, ist dieser, wie aus Figur 3 ersichtlich, elliptisch ausgeführt, so daß die Vorsprünge 10 an der Stelle der größten Ausdehnung der durch den Vorsprung belassenen Öffnung in das Innere des Standbehälters einführbar und durch Drehen in die in Figur 2 dargestellte Lage gebracht werden können, in welcher der Nadelkonus formschlüssig gesichert ist.

## Patentansprüche

1. Vorrichtung zur Aufnahme von benutzten Punktionsnadeln oder Injektionsnadeln **gekennzeichnet durch** einen einseitig offenen, röhrenförmigen Schutzbehälter (2) aus einem gegen Durchstechen widerstandfähigen Material mit einem Innenraum zur vollständigen Aufnahme der gesamten Punktionsnadel oder Injektionsnadel, der an in seinem oberen offenen Bereich einen dem Nadelkonus (8) angepaßten Aufnahmekonus (2a) zur reibschlüssigen Festlegung des Nadelkonus (8) aufweist und **gekennzeichnet durch** einen den röhrenförmigen Schutzbehälter (2) aufnehmenden Standbehälter (3) sowie ferner **gekennzeichnet durch** einen Verschlußkörper (12), der zumindest den Zugang zum Nadelkonus (8) versperrt.

2. Vorrichtung nach Anspruch 1, **dadurch gekennzeichnet, daß** der Standbehälter (3) den röhrenförmigen Schutzbehälter (2) vollständig in sich aufnimmt.

3. Vorrichtung nach Anspruch 1 oder 2, **dadurch gekennzeichnet, daß** der Standbehälter (3) aus einem weichelastischen Kunststoff besteht und durch Umspritzen des Schutzbehälters (2) fest mit diesem verbunden ist.

4. Vorrichtung nach einem der Ansprüche 1 bis 3, **dadurch gekennzeichnet, daß** der Standbehälter (3) über eine angeformte biegsame Lasche (13) den Verschlußkörper (12) trägt.

5. Vorrichtung nach einem der Ansprüche 1 bis 4, **dadurch gekennzeichnet, daß** der Verschlußkörper (12) in den hohlen Nadelkonus (8) einsteckbar ist.

6. Vorrichtung nach einem der Ansprüche 1 bis 4, **dadurch gekennzeichnet, daß** der Verschlußkörper als feststeckbarer Deckel für den Standbehälter (3) ausgebildet ist.

7. Vorrichtung nach einem der Ansprüche 1 bis 6, **dadurch gekennzeichnet, daß** der Standbehälter (3) im Bereich seines oberen offenen Endes mindestens einen Vorsprung (11) für mindestens einen gewindeartigen oder bajonettverschlußartigen Vorsprung (10) am Nadelkonus (8) aufweist.

8. Vorrichtung nach einem der Ansprüche 1 bis 7, **dadurch gekennzeichnet, daß** der Standbehälter (3) einen saugnapfartigen Standfuß (4) aufweist.

9. Vorrichtung nach einem der Ansprüche 1 bis 7, **dadurch gekennzeichnet, daß** der Standbehälter (3) einen unten ebenen Standfuß mit einer Klebebeschichtung aufweist.

10. Vorrichtung nach einem der Ansprüche 1 bis 9, **dadurch gekennzeichnet, daß** der Schutzbehälter (2) eine Behälterwand mit insgesamt konischem Verlauf aufweist, deren Konuswinkel im wesentlichen demjenigen des Nadelkonus (8) entspricht.

11. Vorrichtung nach einem der Ansprüche 1 bis 10, **dadurch gekennzeichnet, daß** der Schutzbehälter (2) an seinem oberen offenen Ende mit einem Außenflansch (7) versehen ist.

12. Vorrichtung nach einem der Ansprüche 1 bis 11, **dadurch gekennzeichnet, daß** der Schutzbehälter (2) aus einem harten Kunststoff besteht.
